# EUROPEAN PATENT APPLICATION

(11) **EP 3 875 560 A1**
(43) Date of publication of application: **08.09.2021**
(21) Application number: 19879085.9
(22) Date of filing: 29.10.2019
(51) Int. Cl.: C09J 183/04, A61F 13/02, A61K 9/70, A61K 47/34, A61L 15/26, C08G 77/38, C09J 7/38, C09J 11/06, C09J 183/06

(54) **PRODUCTION METHOD FOR SILICONE ADHESIVE COMPOSITION**

(30) Priority: 02.11.2018 JP 2018207030
(71) Applicant: SHIN-ETSU CHEMICAL CO., LTD., Tokyo 100-0004 (JP)
(72) Inventor: KOBAYASHI, Akihiro, Annaka-shi, Gunma 379-0224 (JP); KURODA, Yasuyoshi, Annaka-shi, Gunma 379-0224 (JP); AOKI, Shunji, Annaka-shi, Gunma 379-0224 (JP)
(74) Representative: Cockerton, Bruce Roger
(86) International application number: PCT/JP2019/042269
(87) International publication number: WO 2020/090781

(57) **Abstract**

Provided is a method for easily producing a silicone adhesive that has a reduced amount of remaining silanol groups, contains no aromatic hydrocarbon solvent, and is capable of preventing an impact(s) on the human body as well as skin irritancy as caused by remaining and volatilizing substances. The method includes:
a first step of mixing, under the presence of a basic catalyst,
(A) a polydiorganosiloxane,
(B) an organopolysiloxane whose molar ratio of R¹₃SiO_{0.5} unit/ SiO₂ unit is 0.5 to 1.5, and
(C) an aliphatic hydrocarbon solvent; and

a second step of treating a product obtained in the first step with an organic disilazane compound and/or a silane compound, for the purpose of lowering an amount of silanol groups contained in a solid product obtained after the treatment to a range of 0.002 to 0.03 mol/100 g of the solid product obtained after the treatment.

## Description

### TECHNICAL FIELD

The present invention relates to a method for producing a silicone adhesive composition.

### BACKGROUND ART

Since the main skeleton of a polysiloxane composing a silicone adhesive is comprised of Si-O bonds with a high bond energy, silicone adhesives are superior in heat resistance, cold resistance, weather resistance, electric insulation and chemical resistance. Thus, silicone adhesives are used under severe environments such as those under which, for example, a heat resistant tape, an electric insulation tape, a masking tape for operation and a flame-retardant mica tape are used. Further, silicone adhesives are used for skin application as being breathable and mildly-irritating.

For example, an adhesive tape employing a silicone adhesive is used when manufacturing, processing and/or assembling electronic and electric parts. It is preferred that a silicone adhesive having a heat resistance even after being heated at a temperature higher than 250°C be employed in an adhesive tape used for the abovementioned purposes and an adhesive tape used for, for example, manufacturing, fixing and/or binding electronic and electric parts exposed to high temperatures.

Conventionally, since a known silicone adhesive composition is produced using an aromatic hydrocarbon solvent such as toluene and xylene, it was inevitable that during a process for producing an adhesive tape, sheet or label, using these silicone adhesive compositions, a tiny amount of an aromatic solvent will remain in an adhesive layer even after a heating step.

When using a silicone adhesive tape with a tiny amount of an aromatic hydrocarbon remaining therein in a closed space during a production step of an electric and electronic part(s), there may be incurred a problem that a volatile substance of the aromatic hydrocarbon will be generated; and yet another problem that if ultraviolet irradiation is performed in a later step, irradiation will not be carried out efficiently as ultraviolet shall be absorbed by the aromatic hydrocarbon.

There are known heretofore a silicone adhesive composition that contains no aromatic hydrocarbon solvent and is produced using, as a raw material, a MQ resin prepared in an aliphatic hydrocarbon solvent; and an adhesive tape, sheet or label coated with such adhesive composition (Patent document 1).

However, a problem with the above method is that when an amine compound and/or alkaline impurities are contained in, for example, an adhesive tape, sheet or label, these adhesive tape, sheet or label will gelate or exhibit a higher viscosity with time. This is because the remaining silanol groups in the silicone adhesive shall react with the amine compound, or the remaining silanol groups themselves in the silicone adhesive shall undergo condensation with the alkaline impurities acting as catalysts. In this regard, it is desired that the amount of the remaining silanol groups contained in a silicone adhesive be reduced in order to inhibit gelation and an increase in viscosity with time.

Further, a silicone adhesive is often used in an adhesive tape or label that is used for e.g. architectural and interior purposes as the surface of an adherend may often contain a silicone resin or a fluorine resin, or be that treated with silicone or fluorine, for e.g. water repelling, oil repelling and dirt prevention purposes. As for adhesive tapes or labels used in a living environment, such as an adhesive paper for pasting a wallpaper, an adhesive paper for attaching a veneer or the like of a piece of furniture etc., a water-proof/air-proof adhesive tape, an adhesive tape for interior fixation, a tape used for repairs, a decoration fixation tape, an adhesive tape for preventing glass splattering, a light shielding adhesive film and an automobile interior fixation tape, when an aromatic hydrocarbon solvent has remained, volatilized into and then stayed in the room air, it may constitute causes of, for example, chemical allergy, sick house syndrome and chemical sensitivity even at a tiny amount.

In addition, silicone adhesives are used as, for example, adhesives for transdermal formulations and adhesive skin patches in terms of medicinal uses, adhesives for medical tapes such as dressing tapes and surgical tapes in terms of medical equipment uses, and adhesives for skin fixation required for wearable devices in terms of a use other than the aforementioned uses.

However, it is known that when using an amino functional group-containing drug as a drug substance of a transdermal formulation, this amino functional group-containing drug shall act as a catalyst for promoting the reaction of the remaining silanol groups in a silicone adhesive such that a silicone adhesive material will exhibit an improved shear property and an impaired adhesion property such as tackiness (Patent document 2).

As a method for reducing the amount of the remaining silanol groups contained in a silicone adhesive, there is disclosed, for example, a method for reducing the contained amount of the remaining silanol groups by reacting the silicone adhesive with an organo silicone endblocking agent in an aromatic hydrocarbon solvent such as toluene and xylene (Patent documents 3 and 4).

However, since this method uses an aromatic hydrocarbon solvent such as toluene and xylene which are unfavorable to the human body, there exists a possibility that these solvents may remain in a material for skin application and bring about negative effects to the human body, such as skin irritancy.

Further, there is also disclosed a method where after reducing the contained amount of the remaining silanol groups by reacting a silicone adhesive with an organo silicone endblocking agent in an aromatic hydrocarbon solvent such as xylene, a composition obtained is then placed on a release liner, followed by removing the solvent with an oven, and then performing redissolution using an ester organic solvent such as ethyl acetate which is relatively highly safe to a living body (Patent document 5).

However, in this method, since xylene is volatilized and then redissolved in ethyl acetate after being used, a longer production time is required as there are required more steps, which also triggers an increase in cost.

In this way, it is required that there be developed a production method that has no more steps than a conventional art, and is capable of producing a silicone adhesive having a reduced amount of remaining silanol groups, without using an aromatic hydrocarbon solvent such as toluene and xylene that are undesirable to the human body, but using an aliphatic hydrocarbon solvent or ester solvent that is relatively safe to the human body.

### PRIOR ART DOCUMENTS

### Patent document

Patent document 1: Japanese Patent No.4648011
Patent document 2: U.S. Reissue Patent No.35474
Patent document 3: Japanese Examined Patent Application Publication No. Hei 2-36634
Patent document 4: Japanese Examined Patent Application Publication No. Hei 7-88297
Patent document 5: Japanese Patent No.4792152

### SUMMARY OF THE INVENTION

### Problems to be solved by the invention

It is an object of the present invention to provide a method for easily producing a silicone adhesive that has a reduced amount of remaining silanol groups, contains no aromatic hydrocarbon solvent, and is capable of preventing an impact(s) on the human body as well as skin irritancy as caused by remaining and volatilizing substances.

### Means to solve the problems

The inventors of the present invention diligently conducted a series of studies to achieve the above object, and completed the invention as follows. That is, the inventors found that by reacting, in an aliphatic hydrocarbon solvent, a component (D) with a product obtained by mixing part of or all the following components (A) and (B) under the presence of a basic catalyst, there could be obtained a silicone adhesive composition for skin application with remaining silanol groups contained therein being in an amount of a range of 0.002 to 0.03 mol/100 g of a solid product obtained after the treatment with the component (D), without using an aromatic hydrocarbon solvent.

Therefore, the present invention is to provide the following silicone adhesive for skin application; and a method for producing this silicone adhesive for skin application.

[1] A method for producing a silicone adhesive composition, comprising:
   a first step of mixing, under the presence of a basic catalyst,
      (A) 20 to 80 parts by mass of a polydiorganosiloxane,
      (B) 80 to 20 parts by mass of an organopolysiloxane containing a R¹₃SiO_{0.5} unit (R¹ independently represents a monovalent hydrocarbon group having 1 to 10 carbon atoms); a SiO₂ unit; and a siloxane unit having a silicon atom-bonded hydroxyl group(s) and/or a siloxane unit having a silicon atom-bonded alkoxy group(s) having 1 to 6 carbon atoms, wherein a molar ratio of R¹₃SiO_{0.5} unit/ SiO₂ unit is 0.5 to 1.5 (provided that a total amount of the components (A) and (B) is 100 parts by mass), and
      (C) an aliphatic hydrocarbon solvent; and
   a second step of treating a product obtained in the first step with (D) an organic disilazane compound represented by the following general formula (1) and/or a silane compound represented by the following general formula (2), for the purpose of lowering an amount of silanol groups contained in a solid product obtained after the treatment to a range of 0.002 to 0.03 mol/100 g of the solid product obtained after the treatment,

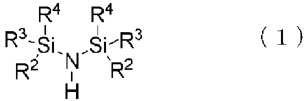

      R²R³R⁴SiCl (2)

      wherein each of R², R³ and R⁴ independently represents a monovalent hydrocarbon group having 1 to 10 carbon atoms and containing no aliphatic unsaturated bonds.
[2] The method for producing the silicone adhesive composition according to [1], wherein the component (A) is a polydiorganosiloxane represented by the following general formula (a): 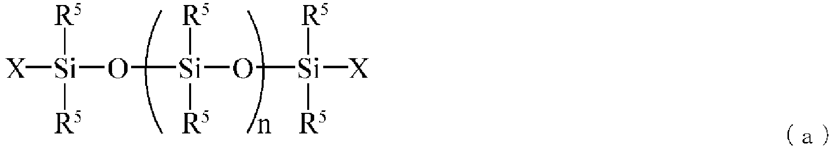 wherein each R⁵ independently represents a monovalent hydrocarbon group having 1 to 10 carbon atoms, X represents OH, n represents a number satisfying 100≤n≤20,000.
[3] The method for producing the silicone adhesive composition according to [1] or [2], wherein the component (C) is at least one selected from hexane, heptane, isooctane, octane, nonane, decane, undecane, dodecane, tridecane, tetradecane, cyclohexane, methylcyclohexane and isoparaffin.
[4] The method for producing the silicone adhesive composition according to any one of [1] to [3], wherein (E) trifluoroacetic acid is used as a reaction catalyst in the second step.
[5] The method for producing the silicone adhesive composition according to any one of [1] to [4], wherein the basic catalyst is an ammonia water.
[6] The method for producing the silicone adhesive composition according to any one of [1] to [5], wherein the silicone adhesive composition is for skin application.
[7] A method for producing a silicone adhesive tape for skin application, comprising:
   applying a silicone adhesive composition for skin application that is produced by the production method according to [6] to a base material.

### Effects of the invention

Since the method of the present invention is a method capable of easily producing a silicone adhesive that has a reduced amount of remaining silanol groups and contains no aromatic hydrocarbon solvent, the method is useful in producing, for example, a silicone adhesive for skin application that is required to be able to prevent an impact(s) on the human body as well as skin irritancy as caused by remaining and volatilizing substances; and a silicone adhesive tape for skin application that utilizes such adhesive.

### MODE FOR CARRYING OUT THE INVENTION

The present invention is described in detail hereunder.

### <Component (A)>

A component (A) is a polydiorganosiloxane, preferably represented by the following formula (3). Further, it is preferred that the component (A) be that whose molecular chain end(s) is an alkoxy group having 1 to 6 carbon atoms or a silicon atom-bonded hydroxyl group. 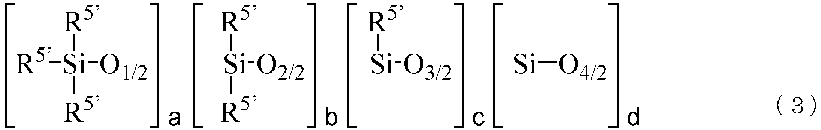 (In this formula, each R^{5'} independently represents a monovalent hydrocarbon group having 1 to 10 carbon atoms, an alkoxy group having 1 to 6 carbon atoms or a hydroxyl group, where at least two of R^{5'}s are an alkoxy group having 1 to 6 carbon atoms or a hydroxyl group. a represents an integer of not smaller than 2, b represents an integer of not smaller than 1, c represents an integer of not smaller than 0, provided that 100≤a+b+c+d≤20,000.)

In the formula (3), each R^{5'} independently represents a monovalent hydrocarbon group having 1 to 10, preferably 1 to 8 carbon atoms; an alkoxy group having 1 to 6 carbon atoms; or a hydroxyl group. Examples of the monovalent hydrocarbon group having 1 to 10 carbon atoms, as represented by R^{5'}, include alkyl groups such as a methyl group, an ethyl group, a propyl group and a butyl group; cycloalkyl groups such as a cyclohexyl group; aryl groups such as a phenyl group and a tolyl group; alkenyl groups such as a vinyl group, an allyl group and a hexenyl group; and even a 3-aminopropyl group, 3,3,3-trifluoropropyl group and 3-hydroxypropyl group that are obtained by substituting a part of or all the carbon atom-bonded hydrogen atoms in the aforementioned groups with other groups. Examples of the alkoxy group having 1 to 6 carbon atoms, as represented by R^{5'}, include a methoxy group, an ethoxy group and a propoxy group. Among the abovelisted examples of R^{5'}, a methyl group or a phenyl group is preferred, where a methyl group is particularly preferred. In the case of phenyl group, it is preferred that phenyl groups be contained in an amount of 0.1 to 30 mol% with respect to all the silicon atom-bonded organic groups in the polydiorganosiloxane represented by the formula (3). When such contained amount is greater than 30 mol%, a silicone adhesive layer obtained will exhibit an impaired adhesive property (tackiness, adhesive force), which makes the adhesive less qualified as an adhesive for skin application.

While a represents an integer of not smaller than 2, b represents an integer of not smaller than 1, it is preferred that each of a and b represents 100 to 20,000; c represents an integer of not smaller than 0, d represents an integer of not smaller than 0; a, b, c and d satisfy 100≤a+b+c+d≤20,000, preferably 150≤a+b+c+d≤15,000. When a+b+c+d is smaller than 100, the composition will exhibit an extremely low viscosity, which may make it difficult to perform coating in a uniform manner. Further, when a+b+c+d is larger than 20,000, the composition will exhibit an extremely high viscosity, which may lead to a poor workability as, for example, it is now difficult to perform stirring and mixing.

The property of the polydiorganosiloxane as the component (A) may be that of an oil or a crude rubber. It is preferred that the viscosity of the component (A) at 25°C be not lower than 300 mPa·s, particularly preferably 3,000 to 1,000,000 mPa·s, provided that the component (A) is in the form of an oil. Further, if the component (A) is in the form of a crude rubber, it is preferred that a solution prepared by dissolving the component (A) in toluene at a concentration of 30% by mass have a viscosity of 1,000 to 100,000 mPa·s, particularly preferably 3,000 to 80,000 mPa·s. When the viscosity is lower than the lower limit value, it may be difficult to apply the adhesive composition in a uniform manner; when the viscosity is greater than the upper limit value, the adhesive composition will exhibit an excessively high viscosity such that it may be difficult to perform stirring when producing such composition.

Here, in this specification, the viscosity is a value measured by a BM-type rotary viscometer at 25°C (the same applies below).

It is preferred that the alkoxy groups or hydroxyl groups be present in the component (A) by an amount of 0.002 to 0.45% by mass, particularly preferably 0.005 to 0.4% by mass.

The component (A) is normally produced by performing ring-opening polymerization on a monomer such as octamethylcyclotetrasiloxane, using a catalyst. However, since a cyclic low-molecular siloxane will be contained after polymerization, it is preferred that there be employed a component from which this cyclic low-molecular siloxane has already been removed as a result of simultaneously passing an inert gas while performing heating and/or under a reduced pressure.

One kind of the component (A) may be used alone; or two or more kinds thereof may be used in combination.

Specific examples of the component (A) include, but are not limited to the following examples. Here, in the following formulae, specific examples of R⁵ include those similar to the abovementioned monovalent hydrocarbon groups each having 1 to 10 carbon atoms, serving as the examples of R^{5'}; and Me and Ph respectively represent a methyl group and a phenyl group. 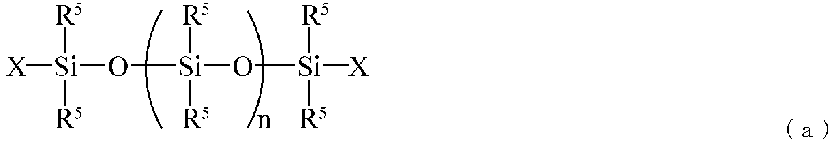 (In this formula, each R⁵ independently represents a monovalent hydrocarbon group having 1 to 10 carbon atoms; X represents OH. n represents a number satisfying 100≤n≤20,000.) 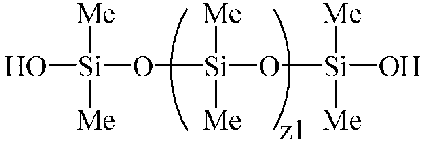 (100≤z1≤20,000) 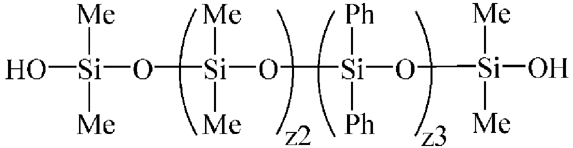 (100≤z2≤19,999, 1≤z3≤2,000, 101≤z2+z3≤20,000)

### <Component (B)>

A component (B) is an organopolysiloxane containing a R¹₃SiO_{0.5} unit (R¹ independently represents a monovalent hydrocarbon group having 1 to 10 carbon atoms); a SiO₂ unit; and a siloxane unit having a silicon atom-bonded hydroxyl group(s) and/or a siloxane unit having a silicon atom-bonded alkoxy group(s) having 1 to 6 carbon atoms, where a molar ratio of R¹₃SiO_{0.5} unit/ SiO₂ unit is 0.5 to 1.5, preferably 0.6 to 1.3. When this molar ratio is lower than 0.5, the silicone adhesive layer obtained will exhibit an impaired adhesive force and tackiness. When this molar ratio is greater than 1.5, the silicone adhesive layer obtained will exhibit an impaired adhesive force and retaining force.

R¹ represents a monovalent hydrocarbon group having 1 to 10, preferably 1 to 8 carbon atoms, specific examples of which include alkyl groups such as a methyl group, an ethyl group, a propyl group and a butyl group; cycloalkyl groups such as a cyclohexyl group; aryl group such as a phenyl group; and alkenyl groups such as a vinyl group, an allyl group and a hexenyl group. Further examples of R¹ may include a 3-aminopropyl group, 3,3,3-trifluoropropyl group and 3-hydroxypropyl group that are obtained by substituting a part of or all the carbon atom-bonded hydrogen atoms in the aforementioned groups with other groups. Among the above examples of R¹, a methyl group is preferred. Further, examples of the alkoxy group having 1 to 6 carbon atoms may include a methoxy group, an ethoxy group and a propoxy group.

Moreover, although the R¹₃SiO_{0.5} unit (M unit) and the SiO₂ unit (Q unit) are essential to the component (B), a R¹SiO_{1.5} unit (T unit) may also be contained in the component (B) so long as the properties of the present invention will not be impaired. However, the component (B) differs from the component (A) in that the component (B) does not have a R¹₂SiO unit (D unit). Here, while there are no particular restrictions on a total amount of the R¹₃SiO_{0.5} unit and SiO₂ unit in the component (B) so long as the properties of the present invention will not be impaired, it is preferred that they are present in an amount of 80 to 100 mol%, more preferably 90 to 100 mol%, with respect to all the siloxane units in the component (B).

The component (B) contains a siloxane unit having a silicon atom-bonded hydroxyl group(s) (silanol group-containing unit); and/or a siloxane unit having a silicon atom-bonded alkoxy group(s) having 1 to 6 carbon atoms (alkoxy group-containing unit). It is preferred that the silanol group-containing unit(s) be present in such an amount that the hydroxyl groups are contained in the component (B) by 0.1 to 5% by mass, more preferably 0.2 to 4% by mass. When the amount of the hydroxyl groups contained is greater than 5% by mass, the silicone adhesive layer obtained may exhibit an impaired tackiness and an impaired curability. When the amount of the hydroxyl groups contained is smaller than 0.1% by mass, an impaired adhesive force may be observed as a later-described condensation reaction between the components (B) themselves or between the components (A) and (B); or a condensation reaction involving a component (C) at the time of curing will be insufficient. Further, it is preferred that the alkoxy group-containing unit(s) be present in such an amount that the alkoxy groups are contained in the component (B) by an amount of not larger than 10% by mass, preferably not larger than 8% by mass. When the amount of the alkoxy groups contained is greater than 10% by mass, the silicone adhesive layer obtained may exhibit an impaired tackiness and an impaired curability. In addition, it is preferred that a total amount of the silanol group-containing units and the alkoxy group-containing units, in terms of a total contained amount of the hydroxyl groups and alkoxy groups, be 0.1 to 12% by mass, particularly preferably 0.2 to 10% by mass, with respect to the component (B). Here, examples of the silanol group-containing units include a R¹₂(HO)SiO_{0.5} unit, R¹(HO)₂SiO_{0.5} unit, R¹(HO)SiO unit and (HO)SiO_{1.5} unit. Further, examples of the alkoxy group-containing units include a R¹₂(R'O)SiO_{0.5} unit, R¹(R'O)₂SiO_{0.5} unit, R¹(R'O)SiO unit and (R'O)SiO_{1.5} unit (R'O independently represents an alkoxy group having 1 to 6 carbon atoms, examples of which include those listed above).

It is highly preferable that the component (B) used in the present invention be that produced without using an aromatic hydrocarbon solvent. As a production method thereof, there may be employed, for example, (a) a method having a step of generating a hydrosol in a solvent containing sodium silicate and water and under the presence of an acid, and a step of reacting the hydrosol with triorganohalosilane; (b) a method having a step of adding sodium silicate to a mixed system containing triorganohalosilane and an acid; or (c) a method having a step of reacting tetraalkoxysilane and/or a hydrolysate thereof with hexaorganodisiloxane under the presence of an acid and water. Any one of these methods may be employed.

Specifically, the method (a) is a known method disclosed in US Patents No.2676182 and No.2814601. That is, it is preferred that the method have a step of generating a hydrosol in a solvent containing sodium silicate and water and under the presence of an acid, and then adding an alcohol to stabilize the hydrosol, followed by reacting the hydrosol with triorganohalosilane and then adding an aliphatic hydrocarbon solvent. It is preferred that the method further include a step of separating an organic layer containing a target organopolysiloxane and an aqueous layer from each other after the reaction is over, removing an alcohol and acid remaining in the organic layer via water washing if necessary, and/or neutralizing them with a base. Moreover, if necessary, there may also be added a step of removing a remaining water, performing condensation with a base catalyst, and adding a non-aromatic solvent or the like to adjust the concentration of the organopolysiloxane. In addition, there may further be added a step of carrying out powderization using a spray dryer or the like.

Specifically, the method (b) is a known method disclosed in JP-A-2003-213000. That is, it is preferred that the method include a step of adding an oxygen-containing solvent having a water solubility of 1 to 100 at room temperature to a mixed system containing triorganohalosilane and an acid, and then adding a mixture of sodium silicate and water to cause reaction. It is preferred that the method further include a step of separating an organic layer containing a target organopolysiloxane and an aqueous layer from each other after the reaction is over, removing an alcohol and acid remaining in the organic layer via water washing if necessary, and/or neutralizing them with a base. Moreover, if necessary, there may also be added a step of removing a remaining water, performing condensation with a base catalyst, and adding a non-aromatic solvent or the like to adjust the concentration of the organopolysiloxane. In addition, there may further be added a step of carrying out powderization using a spray dryer or the like.

Specifically, the method (c) is a known method disclosed in JP-A-Sho 61-195129, Japanese Examined Patent Application Publications No. Hei 6-23253 and Hei 6-33335. That is, it is preferred that the method have a step of reacting tetraalkoxysilane and/or a hydrolysate thereof with hexaorganodisiloxane by adding an acid and water. It is preferred that the method further include a step of separating an organic layer containing a target organopolysiloxane and an aqueous layer from each other after the reaction is over, removing an alcohol and acid remaining in the organic layer via water washing if necessary, and/or neutralizing them with a base. Moreover, if necessary, there may also be added a step of removing a remaining water, performing condensation with a base catalyst, and adding a non-aromatic solvent or the like to adjust the concentration of the organopolysiloxane. In addition, there may further be added a step of carrying out powderization using a spray dryer or the like.

It is preferred that the component (B) have a weight-average molecular weight of 500 to 10,000, more preferably 1,000 to 8,000. This weight-average molecular weight can usually be obtained as, for example, a weight-average molecular weight in terms of polystyrene in gel permeation chromatography (GPC) analysis (same as below) using toluene as a developing solvent.

Further, one kind of the component (B) may be used alone; or two or more kinds thereof may be used in combination.

### <Components (A) and (B)>

A compounding ratio i.e. mass ratio between the components (A) and (B) is (A)/(B)=20/80 to 80/20, preferably 25/75 to 70/30, more preferably 30/70 to 60/40. When such compounding ratio is lower than 20/80, the silicone adhesive layer obtained will exhibit an impaired adhesive force and retaining force. Meanwhile, when this compounding ratio is greater than 80/20, the silicone adhesive layer obtained will exhibit an impaired adhesive force and tackiness.

### <Component (C)>

A component (C) is an aliphatic hydrocarbon solvent, examples of which include those capable of uniformly dissolving the components (A) and (B). Specific examples of the component (C) include aliphatic hydrocarbon solvents such as hexane, heptane (n-heptane), octane, isooctane, nonane, decane, undecane, dodecane, tridecane, tetradecane, cyclohexane, methylcyclohexane and isoparaffin; or a mixed solvent(s) thereof. As a commercially available isoparaffin, there may be used, for example, ISOPAR E. The component (C) virtually does not contain an aromatic hydrocarbon solvent such as benzene, toluene, xylene, ethylbenzene and styrene. As the component (C), preferred are hexane, heptane, octane and isoparaffin; more preferred are heptane and isoparaffin.

Here, the expression "virtually does not contain" refers to a state where benzene, toluene, xylene, ethylbenzene, styrene and the like contained in the aromatic hydrocarbon solvent as the component (C) are present at a concentration of not higher than 10 ppm.

It is preferred that the component (C) be added in an amount of 15 to 240 parts by mass, more preferably 25 to 150 parts by mass, per a total of 100 parts by mass of the components (A) and (B).

### <Component (D)>

A component (D) is an organic disilazane compound and/or a silane compound.

The organic disilazane compound is represented by the following general formula (1). 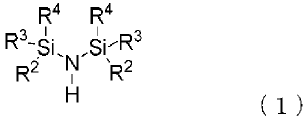 (In this formula, each of R², R³ and R⁴ independently represents a monovalent hydrocarbon group having 1 to 10 carbon atoms and containing no aliphatic unsaturated bonds.)

Each of R², R³ and R⁴ independently represents a monovalent hydrocarbon group having 1 to 10 carbon atoms and containing no aliphatic unsaturated bonds. Examples of the monovalent hydrocarbon group having 1 to 10 carbon atoms and containing no aliphatic unsaturated bonds, include an alkyl group having 1 to 10 carbon atoms; a cycloalkyl group having 3 to 10 carbon atoms; an alkenyl group having 2 to 6 carbon atoms; and a phenyl group. Specifically, as the alkyl group having 1 to 10 carbon atoms, there can be listed alkyl groups such as a methyl group, ethyl group, propyl group, butyl group, pentyl group, hexyl group, heptyl group, octyl group, nonyl group and decyl group; as the cycloalkyl group having 3 to 10 carbon atoms, there can be listed cycloalkyl groups such as a cyclopropyl group, cyclobutyl group, cyclopentyl group and cyclohexyl group. As the alkenyl group having 2 to 6 carbon atoms, there may be listed, for example, a vinyl group, allyl group, 1-butenyl group, 2-butenyl group and 2-pentenyl group. As R², R³ and R⁴, preferred are a methyl group, ethyl group, vinyl group and phenyl group.

Examples of specific structures of the organic disilazane compound include, but are not limited to the following structures. In the following formulae, Me, Et, Vi and Ph respectively represent a methyl group, an ethyl group, a vinyl group and a phenyl group.

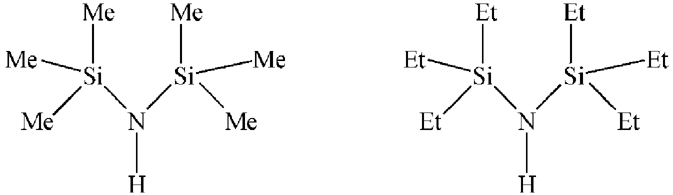

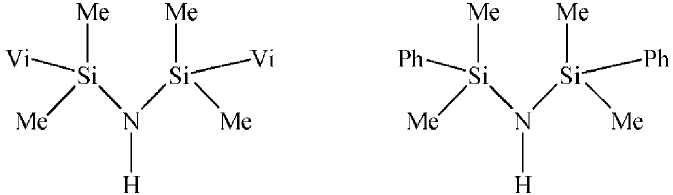

While an ammonia gas will be generated as a by-product as a result of having the silanol groups of a product obtained in a first step react with organic disilazane, unlike a halogenated hydrogen generated as a by-product when using a halogenated silane, ammonia shall not corrode a reaction furnace due to its basicity and can be easily removed from the reaction system via heating and refluxing.

The silane compound as the component (D) is represented by the following general formula (2).

R²R³R⁴iCl (2)

(In this formula, each of R², R³ and R⁴ independently represents a monovalent hydrocarbon group having 1 to 10 carbon atoms and containing no aliphatic unsaturated bonds.)

The silane compound refers to chlorosilanes such as trimethylchlorosilane, triethylchlorosilane, triisopropylchlorosilane, dimethylethylchlorosilane, dimethylisopropylchlorosilane, dimethyl-n-propylchlorosilane, triphenylchlorosilane, octyldimethylchlorosilane and allyldimethylchlorosilane, among which trialkylchlorosilanes such as trimethylchlorosilane and triethylchlorosilane are preferred, and more preferred is trimethylchlorosilane.

The organic disilazane compound and/or silane compound as the component (D) is used in an amount of 0.05 to 5 mol, preferably 0.075 to 3.75 mol, more preferably 0.1 to 2.5 mol, per 1 mol of remaining silanol groups of the product obtained in the first step. When the amount of the component (D) used is smaller than 0.05 mol, the amount of the remaining silanol groups cannot be reduced to a given range; when the amount of the component (D) used is greater than 5 mol, the silicone adhesive layer obtained will exhibit an impaired adhesive property, which makes the adhesive less qualified as an adhesive for skin application.

### <Component (E)>

A component (E) is trifluoroacetic acid. If using the component (E), it may simply be added in a catalytic amount e.g. 0.1 to 10 mol%, preferably 0.25 to 8 mol%, more preferably 0.5 to 5 mol%, with respect to the component (D). When the amount of the component (E) is smaller than 0.1 mol%, there cannot be expected the effect of reducing the amount of the remaining silanol groups; when the amount of the component (E) is larger than 10 mol%, the silicone adhesive layer obtained will exhibit an impaired adhesive property, which may make the adhesive less qualified as an adhesive for skin application.

### <Production method of composition>

A production method of the silicone adhesive for skin application in the present invention is characterized by having the following first step and second step.

### <First step>

A product obtained in a first step of the present invention is a condensation reaction product of the components (A) and (B), and can be obtained by mixing the components (A), (B) and (C) under the presence of a basic catalyst either at room temperature or under a heated condition. The condensation reaction product may contain a reaction product generated by a partial condensation of the components (A) and (B), and may contain an excess component(s) of the components (A) and (B). Further, the condensation reaction product may also contain an unreacted product of the components (A) and (B). There are no particular restrictions on an order in which the components are mixed.

Examples of the basic catalyst include metallic hydroxides such as lithium hydroxide, sodium hydroxide, potassium hydroxide and calcium hydroxide; carbonates such as sodium carbonate and potassium carbonate; hydrogen carbonates such as sodium hydrogen carbonate and potassium hydrogen carbonate; metallic alkoxides such as sodium methoxide and potassium butoxide; organic metals such as butyllithium; potassium silanolate; and nitrogen compounds such as an ammonia gas, an ammonia water, methylamine, trimethylamine and triethylamine, among which an ammonia gas or an ammonia water is preferred.

While a reaction temperature in the first step may be 10 to 150°C, the reaction may normally be performed at room temperature (25°C) to a reflux temperature of an aliphatic hydrocarbon solvent. Although there are no particular restrictions on a reaction time, the reaction time may be 0.5 to 20 hours, preferably 1 to 16 hours.

Further, after the reaction is over, there may be added a neutralizer for neutralizing the basic catalyst, if necessary. Examples of the neutralizer include acid gases such as a hydrogen chloride gas and a carbon dioxide gas; organic acids such as acetic acid, octylic acid and citric acid; and mineral acids such as hydrochloric acid, sulfuric acid and phosphoric acid. If using an ammonia gas, ammonia water or low-boiling amine compound as the basic catalyst, such catalyst may be removed by passing an inert gas such as a nitrogen gas.

### <Second step>

A product obtained in a second step of the present invention is produced by adding the component (D) to the product obtained in the first step and causing a reaction thereby. Here, if adding the trifluoroacetic acid as the component (E), the trifluoroacetic acid is added after the component (D) was added.

While a reaction temperature in the second step may be 10 to 120°C, it is preferred that this reaction temperature be 25 to 90°C, more preferably 40 to 80°C. Although there are no particular restrictions on a reaction time including a mixing and refluxing time, the reaction time is 0.5 to 20 hours, preferably 1 to 16 hours, more preferably 2 to 10 hours.

Further, if necessary, water may be added after the reaction is over, and it is preferred that water be added in an amount of 1 to 16 parts by mass per the total of 100 parts by mass of the components (A) and (B).

Furthermore, after the reaction is over, there may also be added a neutralizer for neutralizing ammonia generated as a by-product, if necessary. Examples of the neutralizer include acid gases such as a hydrogen chloride gas and a carbon dioxide gas; organic acids such as acetic acid, octylic acid and citric acid; and mineral acids such as hydrochloric acid, sulfuric acid and phosphoric acid. Here, the ammonia may also be removed by passing an inert gas such as a nitrogen gas.

As a method for determining the amount of the silanol groups contained in a silicone adhesive, there are disclosed a number of methods utilizing the chemical reactivity thereof. For example, there is known a method where a substrate (target substance for measuring contained amount of silanol groups) is to be dissolved in a dehydrated toluene, followed by reacting them with a Grignard reagent (CH₃Mgl), and then obtaining the amount of the silanol groups contained by quantifying methane generated by the reaction below.

Here, in this specification, the amount of the silanol groups contained is obtained by the above method.

≡SiOH + CH₃MgI → ≡SiOMgI + CH₄

The amount of the silanol groups (silicon-bonded hydroxyl groups) contained in the product obtained in the second step is in a range of 0.002 to 0.03 mol/100 g of a solid product obtained after the treatment in the second step, preferably 0.003 to 0.025 mol/100 g of the solid product obtained after the treatment in the second step, more preferably 0.004 to 0.02 mol/100 g of the solid product obtained after the treatment in the second step.

Various types of reaction containers may be used to carry out the method described in this specification. For example, in one typical embodiment, the reaction container is a batch or semibatch reactor capable of performing mixing via a stirrer, a stirring paddle or other mixing means. Alternatively, the contents in the reaction container may be mixed via a sequence of mixing loop(s) and a mixing chamber. The reaction container may also be equipped with an inert gas purge. The inert gas purge may contain a nitrogen gas, an argon gas; or other inert gases that are nonreactive with the contents in the reaction container.

### <Use of composition>

A silicone adhesive layer can be obtained by applying the silicone adhesive composition for skin application of the present invention that is produced in the above manner to various base materials, and then performing drying under a given condition.

### <Adhesive tape for skin application>

For example, the composition may be favorably used as an adhesive tape for skin application having a base material; and an adhesive layer comprised of a cured product of the silicone adhesive composition for skin application, the adhesive layer being provided on at least one surface of the base material.

Examples of the base material include a paper, a plastic film, an unwoven cloth, a laminating film, a glass, a metal and a cloth. Examples of a paper include a high-quality paper, a coated paper, an art paper, a glassine paper, a polyethylene laminating paper, a kraft paper, a Japanese paper and a synthetic paper. Examples of a plastic film include a polyethylene film, a polypropylene film, a polyester film, a polyimide film, a polyamide film, a polyurethane film, a polyurea film, a polyvinyl chloride film, a polyvinylidene chloride film, a polyvinyl alcohol film, a polycarbonate film, a polytetrafluoroethylene film, a polystyrene film, an ethylene-vinyl acetate copolymer film, an ethylene-vinyl alcohol copolymer film, a triacetyl cellulose film, a polyether ether ketone film and a polyphenylene sulfide film. Examples of an unwoven cloth include a synthetic fiber unwoven cloth and a natural fiber unwoven cloth. Examples of a laminating film include those produced by laminating an unwoven cloth and a plastic film; and those produced by laminating a paper and a plastic film. As for a glass, there are no particular restrictions on, for example, a thickness and type thereof, and a chemically strengthened glass may, for example, be used. Further, a glass fiber may also be employed; the glass fiber may be used alone or combined with other resins before use. Examples of a metal include an aluminum foil, a copper foil, a gold foil, a silver foil and a nickel foil.

In order to further improve an adhesiveness between these base materials and the silicone adhesive layer, the base materials may be those that have been subjected to a primer treatment, corona treatment, etching treatment or plasma treatment.

Coating may be performed by a known method, examples of which include methods using a comma coater, a lip coater, a roll coater, a die coater, a knife coater, a blade coater, a rod coater, a bar coater, a kiss coater or a gravure coater; and methods such as screen coating, dip coating and cast coating. Before performing coating, the composition may be appropriately diluted with a solvent described above as an optional component. In such case, there may be used a solution of the silicone adhesive composition that has a concentration of 20 to 80% by mass.

A coating amount is appropriately determined depending on the intended use; normally, it is preferred that the coating amount be such an amount that a thickness of the silicone adhesive layer after curing will be 2 to 2,000 µm, particularly 3 to 1,000 µm.

As a curing condition of the silicone adhesive composition, the composition may be treated at 70 to 250°C for 10 sec to 10 min. However, the curing condition is not limited to this condition.

The adhesive tape of the present invention may be produced by directly applying the silicone adhesive composition for skin application of the present invention to a base material as above, and then turning the composition into a silicone adhesive layer via curing; or by applying the composition to a release film or release paper that has been subjected to release coating, turning the composition into the silicone adhesive layer via curing, and then pasting such silicone adhesive layer on the above base material by a transfer method.

### WORKING EXAMPLES

The present invention is described in detail hereunder with reference to working and comparative examples. However, the present invention is not limited to the following working examples. Here, in the following examples, unless otherwise noted, "%" in the composition refers to % by mass, and "part(s)" in the composition refers to part(s) by mass.

### <Raw material used>

### Component (A)

### (A-1)

Dimethylpolysiloxane exhibiting a viscosity of 1,400 mPa·s when dissolved in toluene at a concentration of 30% by mass, and having molecular ends blocked by OH groups. 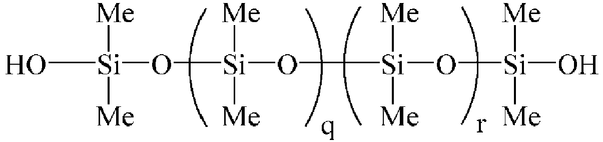 (Here, q and r are numbers satisfying the above viscosity.)

### Component (B)

### (B-1)

A MQ resin having M units of a formula (Me₃SiO_{1/2}) and Q units of a formula (SiO_{4/2}) at a M/Q molar ratio of 0.81/1.0, and having a weight-average molecular weight (Mw) of 2,550, where silanol groups are contained in an amount of 0.11 mol per 100 g of the MQ resin.

### [Working example 1]

A solution comprised of 40 parts of (A-1) as the component (A), 60 parts of (B-1) as the component (B), 42.86 parts of n-heptane and 0.5 parts of an ammonia water was stirred at 25°C for 15 hours. Next, heating was performed at about 100 to 115°C for six hours while performing refluxing, thus removing the ammonia water. After cooling a reaction product obtained to 70°C, 7.35 parts of hexamethyldisilazane were added thereto to then perform heating and stirring at 70°C for four hours, followed by adding 8 parts of water thereto to then perform heating and stirring at 70°C for another 0.5 hours. Later, the temperature was raised to and kept at 100 to 115°C to then perform heating and stirring for six hours while performing refluxing, thereby removing ammonia and water before cooling. In the end, n-heptane was added and mixed therewith so that a solid content would be in an amount of about 70% by mass, thereby synthesizing a silicone adhesive base composition A.

### [Working example 2]

A solution comprised of 40 parts of (A-1) as the component (A), 60 parts of (B-1) as the component (B), 42.86 parts of n-heptane and 0.5 parts of an ammonia water was stirred at 25°C for 15 hours. Next, heating was performed at about 100 to 115°C for six hours while performing refluxing, thus removing the ammonia water. After cooling a reaction product obtained to 70°C, 5.51 parts of hexamethyldisilazane and 2.47 parts of trimethylchlorosilane were added thereto to then perform heating and stirring at 70°C for four hours, followed by adding 8 parts of water thereto to then perform heating and stirring at 70°C for another 0.5 hours. Later, the temperature was raised to and kept at 100 to 115°C to then perform heating and stirring for six hours while performing refluxing, thereby removing ammonia and water before cooling. A byproduct salt was then removed via pressure filtration using a filtration plate NA-10 (by ADVANTEC). In the end, n-heptane was added and mixed therewith so that a solid content would be in an amount of about 70% by mass, thereby synthesizing a silicone adhesive base composition B.

### [Working example 3]

A solution comprised of 40 parts of (A-1) as the component (A), 60 parts of (B-1) as the component (B), 42.86 parts of n-heptane and 0.5 parts of an ammonia water was stirred at 25°C for 12 hours. Next, heating was performed at about 100 to 115°C for six hours while performing refluxing, thus removing the ammonia water. After cooling a reaction product obtained to 60°C, 7.35 parts of hexamethyldisilazane and 0.1 parts of trifluoroacetic acid were added thereto to then perform heating and stirring at 60°C for three hours, followed by adding 8 parts of water thereto to then perform heating and stirring at 70°C for another 0.5 hours. Later, the temperature was raised to and kept at 100 to 115°C to then perform heating and stirring for six hours while performing refluxing, thereby removing ammonia and water before cooling. A byproduct salt was then removed via pressure filtration using a filtration plate NA-10 (by ADVANTEC). In the end, n-heptane was added and mixed therewith so that a solid content would be in an amount of about 70% by mass, thereby synthesizing a silicone adhesive base composition C.

### [Working example 4]

A solution comprised of 40 parts of (A-1) as the component (A), 60 parts of (B-1) as the component (B), 42.86 parts of ISOPAR E (boiling point range 118 to 140°C by MARUZEN CHEMICAL TRADING CO., LTD.) and 0.5 parts of an ammonia water was stirred at 25°C for 15 hours. Next, heating was performed at about 140 to 155°C for six hours while performing refluxing, thus removing the ammonia water. After cooling a reaction product obtained to 70°C, 5.51 parts of hexamethyldisilazane and 2.47 parts of trimethylchlorosilane were added thereto to then perform heating and stirring at 70°C for four hours, followed by adding 8 parts of water thereto to then perform heating and stirring at 70°C for another 0.5 hours. Later, the temperature was raised to and kept at 140 to 155°C to then perform heating and stirring for six hours while performing refluxing, thereby removing ammonia and water before cooling. A byproduct salt was then removed via pressure filtration using a filtration plate NA-10 (by ADVANTEC). In the end, ISOPAR E was added and mixed therewith so that a solid content would be in an amount of about 70% by mass, thereby synthesizing a silicone adhesive base composition D.

### [Comparative example 1]

A solution comprised of 40 parts of (A-1) as the component (A), 60 parts of (B-1) as the component (B), 42.86 parts of xylene and 0.5 parts of an ammonia water was stirred at 25°C for 15 hours. Next, heating was performed at 145 to 160°C for six hours while performing refluxing, thus removing the ammonia water. After cooling a reaction product obtained to 70°C, 7.35 parts of hexamethyldisilazane were added thereto to then perform heating and stirring at 70°C for four hours, followed by adding 8 parts of water thereto to then perform heating and stirring at 70°C for another 0.5 hours. Later, the temperature was raised to and kept at 145 to 160°C to then perform heating and stirring for six hours while performing refluxing, thereby removing ammonia and water before cooling. A product thus obtained was then placed on an appropriate release liner, followed by leaving them in an oven until the xylene solvent had been distilled way, thereby removing the volatile content. In the end, ethyl acetate was added and mixed therewith so that a solid content would be in an amount of about 70% by mass, thereby synthesizing a silicone adhesive base composition E. As a result of measuring a xylene amount by gas chromatography analysis, 35 ppm of remaining xylene was confirmed to be present.

### <Working examples 1 to 4; comparative example 1>

Each of the above silicone adhesive base compositions A to E was individually put into a flask in accordance with compounding ratios (parts) shown in the following table, followed by diluting the same with 23.8 parts of n-heptane, and then stirring and mixing them so as to obtain a silicone adhesive composition. This silicone adhesive composition was then evaluated by the methods below. The results thereof are shown in Tables 1 and 2.

### [Skin adhesive force]

An applicator was used to apply an n-heptane solution of the silicone adhesive composition that has a concentration of 60% by mass to a polyethylene terephthalate film having a thickness of 23 µm and a width of 25 mm in a way such that a thickness of a cured composition would be 30 µm, followed by actually curing the composition at 130°C for 1 min so as to produce an adhesive tape. This adhesive tape was later attached to the forearm of a person and then left at room temperature for about 30 min. Next, at 25°C, one end of such adhesive tape was peeled away using a tensile tester where the adhesive tape was peeled way at a pulling rate of 60 mm/min and an angle of 90°, and measured was a force (N/25 mm) required to peel away the one end of the adhesive tape.

### [Appearance of adhesive tape peeled]

**I**n confirming the skin adhesive force, an appearance of the adhesive tape peeled was observed, and evaluation was performed using a three-level model based on the following criteria.
○: Adhesive layer was barely rough
Δ: Adhesive layer was slightly rough
×: Adhesive layer was rough

### [Adhesive residue on skin after peeling]

In confirming the above adhesive force, observed was an adhesive residue of the adhesive component on the forearm after the adhesive tape was peeled away therefrom, and evaluation was performed using a three-level model based on the following criteria.
○: No adhesive residue
Δ: Slight adhesive residue
×: Mass adhesive residue

### [Durability of adhesive tape]

An applicator was used to apply a 60% by mass n-heptane solution of the silicone adhesive composition to a polyethylene terephthalate film having a thickness of 23 µm and a width of 25 mm in a way such that a thickness of a cured composition would be 30 µm, followed by actually curing the composition at 130°C for 1 min so as to produce an adhesive tape. This adhesive tape was later attached to the forearm of a person. The condition of the test specimen was then observed after 24 hours following the attachment; and evaluation was performed using a three-level model based on the following criteria, in terms of an extent to which a deformation of the test specimen, wrinkles on the test specimen, peeling of the test specimen and an adhesive residue after peeling away the test specimen had occurred.
○: Test specimen barely exhibited deformation, wrinkles, peeling and barely contributed to adhesive residue after peeling.
Δ: Test specimen exhibited deformation, wrinkles, peeling and contributed to adhesive residue after peeling, to a certain extent.
×: Test specimen exhibited deformation, wrinkles, peeling and contributed to adhesive residue after peeling, to a significant extent.

**[Table 1]**

| | Working example | | | |
|---|---|---|---|---|
| | 1 | 2 | 3 | 4 |
| Silicone adhesive base composition A | 142.86 | | | |
| Silicone adhesive base composition B | | 142.86 | | |
| Silicone adhesive base composition C | | | 142.86 | |
| Silicone adhesive base composition D | | | | 142.86 |
| Contained amount of silanol groups [mol/100 g of solid product obtained after treatment in first step] | 0.045 | 0.045 | 0.045 | 0.045 |
| Contained amount of silanol groups [mol/100 g of solid product obtained after treatment in second step] | 0.029 | 0.015 | 0.003 | 0.016 |
| Skin adhesive force [N/25mm] | 2.5 | 2.3 | 2.1 | 2.5 |
| Appearance of adhesive tape peeled | ○ | ○ | ○ | ○ |
| Adhesive residue on skin after peeling | ○ | ○ | ○ | ○ |
| Durability of adhesive tape | ○ | ○ | ○ | ○ |

[Table 2]

| | Comparative example |
|---|---|
| | 1 |
| Silicone adhesive base composition E | 142.86 |
| Contained amount of silanol groups [mol/100 g of solid product obtained after treatment in first step] | 0.048 |
| Contained amount of silanol groups [mol/100 g of solid product obtained after treatment in second step] | 0.028 |
| Skin adhesive force [N/25mm] | 2.4 |
| Appearance of adhesive tape peeled | ○ |
| Adhesive residue on skin after peeling | ○ |
| Durability of adhesive tape | ○ |

In working examples 1 to 4, by using an aliphatic hydrocarbon solvent, a silicone adhesive with a reduced amount of remaining silanol groups was able to be easily produced without using an aromatic hydrocarbon solvent.

Thus, the production method of the present invention is useful for the production of, for example, a silicone adhesive for skin application that is required to be able to prevent an impact(s) on the human body as well as skin irritancy as caused by remaining and volatilizing substances (e.g. aromatic hydrocarbon solvent); and the production of a silicone adhesive tape for skin application using such adhesive.

The present invention is not limited to the above embodiment(s). The above embodiment(s) are listed as examples, and any embodiment substantially having an identical structure, and capable of bringing about similar functions and effects as the technical ideas described in the claims of the present invention shall fall into the technical scope of the present invention.

## Claims

1. A method for producing a silicone adhesive composition, comprising:
a first step of mixing, under the presence of a basic catalyst,
(A) 20 to 80 parts by mass of a polydiorganosiloxane,
(B) 80 to 20 parts by mass of an organopolysiloxane containing a R¹₃SiO_{0.5} unit (R¹ independently represents a monovalent hydrocarbon group having 1 to 10 carbon atoms); a SiO₂ unit; and a siloxane unit having a silicon atom-bonded hydroxyl group(s) and/or a siloxane unit having a silicon atom-bonded alkoxy group(s) having 1 to 6 carbon atoms, wherein a molar ratio of R¹₃SiO_{0.5} unit/ SiO₂ unit is 0.5 to 1.5 (provided that a total amount of the components (A) and (B) is 100 parts by mass), and
(C) an aliphatic hydrocarbon solvent; and
a second step of treating a product obtained in the first step with (D) an organic disilazane compound represented by the following general formula (1) and/or a silane compound represented by the following general formula (2), for the purpose of lowering an amount of silanol groups contained in a solid product obtained after the treatment to a range of 0.002 to 0.03 mol/100 g of the solid product obtained after the treatment, 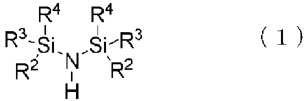
R²R³R⁴SiCl (2)
wherein each of R², R³ and R⁴ independently represents a monovalent hydrocarbon group having 1 to 10 carbon atoms and containing no aliphatic unsaturated bonds.

2. The method for producing the silicone adhesive composition according to claim 1, wherein the component (A) is a polydiorganosiloxane represented by the following general formula (a): 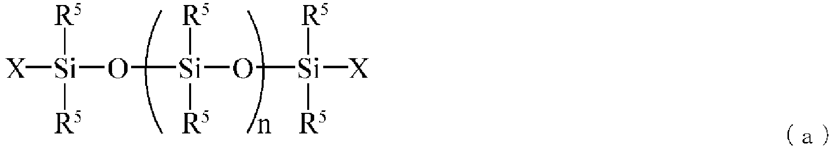 wherein each R⁵ independently represents a monovalent hydrocarbon group having 1 to 10 carbon atoms, X represents OH, n represents a number satisfying 100≤n≤20,000.

3. The method for producing the silicone adhesive composition according to claim 1 or 2, wherein the component (C) is at least one selected from hexane, heptane, isooctane, octane, nonane, decane, undecane, dodecane, tridecane, tetradecane, cyclohexane, methylcyclohexane and isoparaffin.

4. The method for producing the silicone adhesive composition according to any one of claims 1 to 3, wherein (E) trifluoroacetic acid is used as a reaction catalyst in the second step.

5. The method for producing the silicone adhesive composition according to any one of claims 1 to 4, wherein the basic catalyst is an ammonia water.

6. The method for producing the silicone adhesive composition according to any one of claims 1 to 5, wherein the silicone adhesive composition is for skin application.

7. A method for producing a silicone adhesive tape for skin application, comprising:
applying a silicone adhesive composition for skin application that is produced by the production method according to claim 6 to a base material.
